# EUROPÄISCHE PATENTSCHRIFT

(11) **EP 1 361 837 B1**
(45) Veröffentlichungstag und Bekanntmachung des Hinweises auf die Patenterteilung: **11.10.2006**
(21) Anmeldenummer: 02719863.9
(22) Anmeldetag: 18.02.2002
(51) Int. Cl.: A61F 2/30, C25D 3/64, A61L 27/30, C23C 18/00, C23C 16/00

(54) **ENDOPROTHESE MIT GALVANISCHER SILBERSCHICHT**
ENDOPROTHESIS WITH GALVANISED SILVER LAYER
ENDOPROTHESE A COUCHE D'ARGENT GALVANISEE

(30) Priorität: 19.02.2001 DE 10107675
(43) Veröffentlichungstag der Anmeldung: 19.11.2003
(73) Patentinhaber: Implantcast GmbH, 21614 Buxtehude (DE)
(72) Erfinder: GOSHEGER, Georg, 48155 Münster (DE); SASS, Jens, 21614 Buxtehude (DE)
(74) Vertreter: UEXKÜLL & STOLBERG
(86) Internationale Anmeldenummer: PCT/EP2002/001705
(87) Internationale Veröffentlichungsnummer: WO 2002/065953

(56) Entgegenhaltungen:
- EP-A- 0 416 342
- US-A- 3 420 755
- US-A- 4 274 926
- US-A- 4 813 965
- US-A- 5 492 763

## Beschreibung

Die Erfindung betrifft eine Endoprothese mit einer metallischen Trägerstruktur sowie ein Verfahren zum Herstellen einer derartigen Endoprothese.

Eine Endoprothese gemäß dem Oberbegriff von Anspruch 1 ist aus der US-A-4 813 965 bekannt.

Endoprothesen mit einer Trägerstruktur, die teilweise oder vollständig aus Metall besteht, finden in der Medizin vielfältige Anwendungen, insbesondere (aber nicht ausschließlich) auf dem Gebiet des Knochenersatzes. Beispiele sind Gelenkprothesen, die ein nicht mehr funktionsfähiges Gelenk ersetzen sollen, und Baukastensysteme, mit denen Endoprothesen für den Knochenersatz nach Bedarf zusammengesetzt werden können und die vor allem in der Tumorchirurgie verwendet werden.

Beim Einsatz derartiger Endoprothesen lässt sich ein Infektionsrisiko nicht ausschließen. Grundsätzlich ist es zwar möglich, Endoprothesen z.B. mit Antibiotika zu behandeln, aber ein schlechtes Anhaften an der Oberfläche, insbesondere über längere Zeiträume, und unerwünschte Nebenwirkungen sind problematisch.

Aus der US 5 492 763 ist es bekannt, mit Hilfe eines Ionenimplantationsverfahrens Metallatome, wie Silber, Gold, Kupfer, Platin, Iridium, Magnesium und Palladium, in die Oberfläche einer Implantatstruktur einzubringen, die z.B. aus einem Polymermaterial besteht. Dadurch soll eine bakteriostatische oder bakterizide Wirkung erzielt werden. Ionenimplantationsverfahren sind jedoch aufwendig und daher kostspielig.

Es ist Aufgabe der Erfindung, eine kostengünstige und medizinisch unbedenkliche Möglichkeit zu schaffen, um bei Endoprothesen mit einer metallischen Trägerstruktur das Infektionsrisiko zu senken.

Diese Aufgabe wird gelöst durch eine Knochenersatz-Endoprothese mit den Merkmalen des Anspruchs 1 sowie ein Verfahren zum Herstellen einer Knochenersatz-Endoprothese mit den Merkmalen des Anspruchs 3. Vorteilhafte Ausgestaltungen der Erfindung ergeben sich aus den Unteransprüchen.

Die erfindungsgemäße Endoprothese weist eine metallische Trägerstruktur auf, bei der zumindest ein Teil der Oberfläche galvanisch versilbert ist. Insbesondere ist die Endoprothese für den Knochenersatz geeignet.

Es hat sich gezeigt, dass durch eine galvanische Beschichtung mit Silber das Risiko einer Infektion nach der Implantation minimiert wird. Dabei treten keine ernsthaften Nebenwirkungen auf. Eine galvanisch aufgetragene Silberschicht haftet gut an der Trägerstruktur, so dass die infektionshemmende Wirkung der Endoprothese auf lange Zeit erhalten bleibt. Das galvanische Aufbringen einer Silberschicht ist vergleichsweise kostengünstig.

Unter der Silberschicht ist eine Haftschicht vorgesehen, die z.B. Gold aufweisen kann.

Als Metalle für die Trägerstruktur kommt eine Vielzahl von Guss- oder Schmiedelegierungen in Frage. Beispiele sind:
CoCrMo-Gusslegierungen nach ASTM F 75 bzw. ISO 5832-4,
CoCrMo-Schmiedelegierungen nach ISO 5832-12,
CoNiCrMo-Schmiedelegierungen nach ASTM F 562 bzw. ISO 5832-6,
Titan-Schmiedelegierungen nach ISO 5832-2,
TiAlNb-Schmiedelegierungen nach ASTM F 1295-92 bzw. ISO 5832-11,
TiAlV-Schmiedelegierungen nach ASTM F 136 bzw. ISO 5832-3 und
FeCrNiMnMoNbN-Schmiedelegierungen nach ISO 5832-9:

Die Auswahl eines geeigneten Materials für die Trägerstruktur hängt von dem Einsatzgebiet der Endoprothese ab. Auch Endoprothesen mit Metallen, die nicht in der obigen Liste erwähnt sind, können erfindungsgemäß mit einer galvanisch versilberten Oberfläche versehen sein.

Bei dem erfindungsgemäßen Verfahren zum Herstellen einer Knochenersatz-Endoprothese wird eine metallische Trägerstruktur bereitgestellt, und zumindest ein Teil der Oberfläche der Trägerstruktur wird galvanisch versilbert. Vor dem galvanischen Versilbern wird eine Haftschicht, die Gold aufweisen kann, auf die zu versilbernde Oberfläche aufgebracht. Es ist von Vorteil, die Trägerstruktur im Bereich der zu versilbernden Oberfläche vor dem Beschichten zu entoxidieren.

Bei einer vorteilhaften Ausgestaltung des Verfahrens wird eine Trägerstruktur aus einer TiAlV-Schmiedelegierung bereitgestellt. Die zu versilbernde Oberfläche dieser Trägerstruktur wird in einer Vakuumkammer ionisch entoxidiert, und die entoxidierte Oberfläche der Trägerstruktur wird in einer Vakuumkammer ionisch mit Gold beschichtet. Die goldbeschichtete Oberfläche der Trägerstruktur wird dann in einem galvanischen Bad galvanisch versilbert.

Bei einer anderen bevorzugten Ausführungsform des erfindungsgemäßen Verfahrens wird eine Trägerstruktur aus einer CoCrMo-Gusslegierung bereitgestellt, wobei die zu versilbernde Oberfläche der Trägerstruktur in einem galvanischen Bad entoxidiert wird. Die entoxidierte Oberfläche der Trägerstruktur wird in einem galvanischen Bad vergoldet und die goldbeschichtete Oberfläche der Trägerstruktur in einem galvanischen Bad versilbert.

Im Folgenden wird die Erfindung anhand von Ausführungsbeispielen weiter erläutert.

### Beispiel 1

Bei einer Trägerstruktur aus TiAl6V4 nach ISO 5832-3 wird die an der Atmosphäre immer entstehende Oxidschicht in einer ersten Vakuumkammer durch Ionensputtering entfernt (ionisches Entoxidieren). Danach wird die entoxidierte Oberfläche der Trägerstruktur in einer zweiten Vakuumkammer in einem Plasmabeschichtungsverfahren (PVD, physical vapour deposition) ionisch mit Gold beschichtet (Goldbeschuss). Die Goldschicht schützt die Oberfläche vor einer erneuten Oxidation, so dass die behandelte Trägerstruktur problemlos transportiert werden kann. Abschließend wird in einem galvanischen Bad auf die vergoldete Oberfläche eine Silberschicht aufgebracht.

### Beispiel 2

Bei einer Trägerstruktur aus Edelstahl oder einer CoCrMo-Gusslegierung nach ISO 5832-4 wird die oberflächliche Oxidschicht durch galvanische Bäder entfernt. Im gleichen galvanischen Bad kann anschließend eine Goldschicht aufgebracht werden. Die vergoldete Oberfläche wird danach in einem anderen Becken galvanisch versilbert.

## Patentansprüche

1. Knochenersatz-Endoprothese, mit einer, metallischen Trägerstruktur aus einer Guss- oder Schmiedelegierung, **dadurch gekennzeichnet, daß** zumindest ein Teil der Oberfläche galvanisch versilbert ist, und daß unter der Silberschicht eine Haftschicht vorgesehen ist, die vorzugsweise Gold aufweist, wobei an der Oberfläche der Knochenersatz-Endoprothese kein Gold exponiert ist.

2. Knochenersatz-Endoprothese nach Anspruch 1, **dadurch gekennzeichnet, dass** die Trägerstruktur mindestens eines der aus der folgenden Gruppe ausgewählten Materialien aufweist:
CoCrMo-Gusslegierungen, CoCrMo-Schmiedelegierungen, CoNiCrMo-Schmiedelegierungen, Titan-Schmiedelegierungen, TiAlNb-Schmiedelegierungen, TiAlV-Schmiedelegierungen, FeCrNiMnMoNbN- Schmiedelegierungen.

3. Verfahren zum Herstellen einer Knochenersatz-Endoprothese nach Anspruch 1 oder 2, mit den Schritten:
- Bereitstellen einer metallischen Trägerstruktur aus einer Guss- oder Schmiedelegierung,
- Aufbringen einer Haftschicht, die vorzugsweise Gold aufweist, auf zumindest einen Teil der Oberfläche der Trägerstruktur,
- galvanisches Versilbern des mit der Haftschicht versehenen Teils der Oberfläche der Trägerstruktur.

4. Verfahren nach Anspruch 3, **dadurch gekennzeichnet, dass** die Trägerstruktur im Bereich der zu versilbernden Oberfläche vor dem Beschichten entoxidiert wird.

5. Verfahren nach Anspruch 3, mit den Schritten:
- Bereitstellen einer Trägerstruktur aus einer TiAlV-Schmiedelegierung,
- ionisches Entoxidieren der zu versilbernden Oberfläche der Trägerstruktur in einer Vakuumkammer,
- ionisches Goldbeschichten der entoxidierten Oberfläche der Trägerstruktur in einer Vakuumkammer,
- galvanisches Versilbern der goldbeschichteten Oberfläche der Trägerstruktur in einem galvanischen Bad.

6. Verfahren nach Anspruch 3, mit den Schritten:
- Bereitstellen einer Trägerstruktur aus einer CoCrMo-Gusslegierung,
- Entoxidieren der zu versilbernden Oberfläche der Trägerstruktur in einem galvanischen Bad,
- galvanisches Vergolden der entoxidierten Oberfläche der Trägerstruktur in einem galvanischen Bad,
- galvanisches Versilbern der goldbeschichteten Oberfläche der Trägerstruktur in einem galvanischen Bad.

## Claims

1. Bone replacement endoprosthesis, having a metallic support structure made from a cast or wrought alloy, **characterised in that** at least part of the surface is electroplated with silver and **in that** a bonding layer, which preferably includes gold, is provided beneath the layer of silver, without any gold being exposed at the surface of the bone replacement endoprosthesis.

2. Bone replacement endoprosthesis according to claim 1, **characterised in that** the support structure includes at least one material selected from the following group: cast CoCrMo alloys, wrought CoCrMo alloys, wrought CoNiCrMo alloys, wrought titanium alloys, wrought TiAlNb alloys, wrought TiAlV alloys, wrought FeCrNiMnMoNbN alloys.

3. Process for producing a bone replacement endoprosthesis according to either claim 1 or claim 2, comprising the steps of providing a metallic support structure made from a cast or wrought alloy, applying a bonding layer, which preferably includes gold, to at least part of the surface of the support structure, electroplating with silver that part of the surface of the support structure which has been provided with the bonding layer.

4. Process according to claim 3, **characterised in that** the support structure is deoxidised in the region of the surface which is to be plated with silver prior to the coating operation.

5. Process according to claim 3, comprising the steps of providing a support structure made from a wrought TiA1V alloy, ionically deoxidising the surface of the support structure, which is to be plated with silver, in a vacuum chamber, ionically coating the deoxidised surface of the support structure with gold in a vacuum chamber, electroplating the gold-coated surface of the support structure with silver in an electroplating bath.

6. Process according to claim 3, comprising the steps of: providing a support structure made from a cast CoCrMo alloy, deoxidising the surface of the support structure, which is to be plated with silver, in an electroplating bath, electroplating the deoxidised surface of the support structure with gold in an electroplating bath, electroplating the gold-coated surface of the support structure with silver in an electroplating bath.

## Revendications

1. Endoprothèse de remplacement d'os, avec une structure porteuse métallique formée d'un alliage de fonderie ou de forge, **caractérisée en ce qu'**au moins une partie de la surface est argentée par galvanisation et **en ce qu'**on prévoit sous la couche d'argent une couche adhésive, qui présente de préférence de l'or, l'or n'étant pas exposé à la surface de l'endoprothèse de remplacement d'os.

2. Endoprothèse de remplacement d'os selon la revendication 1, **caractérisée en ce que** la structure porteuse présente au moins l'un des matériaux choisis dans le groupe suivant : alliages de fonderie CoCrMo, alliages de forge CoCrMo, alliages de forge CoNiCrMo, alliages de forge de titane, alliages de forge TiAlNb, alliages de forge TiAlV et alliages de forge FeCrNiMnMoNbN.

3. Procédé pour fabriquer une endoprothèse de remplacement d'os selon la revendication 1 ou 2, comprenant les étapes suivantes :
- préparer une structure porteuse métallique à partir d'un alliage de fonderie ou de forge,
- appliquer une couche adhésive, qui présente de préférence de l'or, sur au moins une partie de la surface de la structure porteuse, et
- argenter par galvanisation la partie de la surface de la structure porteuse pourvue de la couche adhésive.

4. Procédé selon la revendication 3, **caractérisé en ce que** la structure porteuse est désoxydée avant revêtement dans la zone de la surface à argenter.

5. Procédé selon la revendication 3, comprenant les étapes suivantes :
- préparer une structure porteuse à partir d'un alliage de forge TiAlV,
- désoxyder par voie ionique la surface à argenter de la structure porteuse dans une chambre à vide,
- revêtir d'or par voie ionique la surface désoxydée de la structure porteuse dans une chambre à vide,
- argenter par galvanisation la surface revêtue d'or de la structure porteuse dans un bain galvanique.

6. Procédé selon la revendication 3, comprenant les étapes suivantes :
- préparer une structure porteuse à partir d'un alliage de fonderie CoCrMo,
- désoxyder la surface à argenter de la structure porteuse dans un bain galvanique,
- dorer par galvanisation ionique la surface désoxydée de la structure porteuse dans un bain galvanique, et
- argenter par galvanisation la surface revêtue d'or de la structure porteuse dans un bain galvanique.
